# EUROPEAN PATENT APPLICATION

(11) **EP 3 435 659 A1**
(43) Date of publication of application: **30.01.2019**
(21) Application number: 17183175.3
(22) Date of filing: 26.07.2017
(51) Int. Cl.: H04N 5/44, H04N 5/45, H04N 5/57, H04N 21/44, H04N 21/431, H04N 21/462, H04N 21/485, A61B 6/00, A61B 8/00, A61B 90/00, H04N 5/46

(54) **AUTOMATIC VIDEO SOURCE DETECTION**

(71) Applicant: Koninklijke Philips N.V., 5656 AE Eindhoven (NL)
(72) Inventor: FRANKE, Nico, 5656 AE Eindhoven (NL)
(74) Representative: de Haan, Poul Erik

(57) **Abstract**

The present invention relates to a system (100) for automatic video source detection, comprising: at least one video input interface (106), configured to for removably connect at least one video source (104), at least one display device (102) for displaying video feeds of different types from the at least one video sources, a memory (108) comprising at least one set of display settings associated with each type of video feed, a processor (110), configured to recognize the type of at least one of the video feeds provided by at least one of the connected video sources based on the contents of the at least one of the video feeds, and to apply display settings to the at least one of the video feeds of the connected video sources based on the recognized type of the at least one of the video feeds. The present invention also relates to a method (200) for automatic video source detection, comprising the steps of: recognizing (202) a type of at least one video feed of at least one video source connected to a system via at least one video input interface, based on the contents of the at least one of the video feeds, and applying (204) display settings associated with each type of video feed to the at least one of the video feeds based on the recognized type of the at least one of the video feeds. Furthermore, the invention relates to a computer program product including a program for a processing device, comprising software code portions for performing the steps of the method when the program is run on the processing device.

## Description

### FIELD OF THE INVENTION

The invention generally relates to display of video signals, and more specifically to automatic detection of provided video feeds.

### BACKGROUND OF THE INVENTION

In systems with a display of video feeds, one or more video sources are connected to a computer or workstation, and the video source is then configured to match user preferences, parameters imposed by the hardware or other criteria. Such system can use various devices for video input (cameras, computers, video streams, etc.) and for video output (monitors, screens, etc.). In particular, in a medical setting, several video sources representing, for example, different imaging sources, may be displayed on one large overhead display. An example of such system is the Philips FlexVision XL display system for the interventional suite.

In order to provide time-crucial operation of these systems, video feeds can be configured differently and consequently displayed with predetermined settings. That way medical personnel can immediately learn certain important information from a screen. For example, different brightness and contrast levels can be automatically applied to different sources.

A problem exists when video sources are added or exchanged. In the above settings, a technical person needs to set up the system after the new or replacement video source is added in order to ensure proper functionality. This even is necessary after an already known video source is re-connected to a system, but at a different connection port.

### SUMMARY OF THE INVENTION

In view of the above-, it would be advantageous to provide a system and a method for automatic video source detection.

In a first aspect, a system for automatic video source detection with at least one video input interface is provided. At least one video source can be removably connected to the system via the at least one video interface. The system further has at least one display device for displaying video feeds of different types from the at least one video source. A memory in the system has stored at least one set of display settings associated with each type of video feed. In the system, there is also a processor, which is configured to recognize the type of at least one of the video feeds provided by at least one of the connected video sources based on the contents of the at least one of the video feeds. The processor is also configured to apply display settings to the at least one of the video feeds of the connected video sources based on the recognized type of the at least one of the video feeds.

In an embodiment, the processor is configured to recognize the type of the at least one of the video feeds in response to a video source being connected to one of the video input interfaces a user request, a hardware request, or a software request.

In a further embodiment, the processor is configured to recognize the type of video feed by using a FLANN, fast approximate nearest neighbor search, image processing algorithm, as is for example described in an article by M. Muja and D.G. Lowe: "Fast approximate nearest neighbors with automatic algorithm configuration", as retrieved from http://www.cs.ubc.ca/~lowe/papers/09muja.pdf on 25 July 2017.

In a further embodiment, the display settings can be one or more of an image for identification, a name for identification, video processing settings, priority settings, position information, size information, orientation information, and device control options.

In a further embodiment, the system is a medical viewing system. For example, the automatic video detection may be incorporated in a Philips FlexVision XL system, which includes a medical-grade 58-inch display monitor with 8 Megapixel display resolution, and an image interface for up to 16 different imaging sources, from which up to 8 can be displayed at once on the monitor.

In a second aspect, a method for automatic video source detection is provided with the steps of recognizing a type of at least one video feed of at least one video source connected to a system via at least one video input interface, based on the contents of the at least one of the video feeds, and applying display settings associated with each type of video feed to the at least one of the video feeds based on the recognized type of the at least one of the video feeds.

In one embodiment, the recognizing is performed in response to one of the video sources is connected to the system, a request of a user of the system, a request of a hardware of the system or connected to the system, or a request of a software on the system.

In a further embodiment, the processor recognizes the type of video feed by using a FLANN, fast approximate nearest neighbor search, image processing algorithm.

These and other aspects of the present invention will become apparent from reference to the embodiments described hereinafter.

### BRIEF DESCRIPTION OF THE DRAWINGS

Exemplary embodiments of the invention will be described in the following with reference to the following drawings:
Fig. 1 shows a schematic drawing of a system for automatic video source detection.
Fig. 2 shows a schematic flowchart of a method for automatic video source detection.
Fig. 3 shows another schematic flowchart of a method for automatic video source detection.

### DETAILED DESCRIPTION OF EMBODIMENTS

Systems for automatic video source detection can be a system for display of several video feeds, such as the above mentioned Philips FlexVision XL. Such a system makes it possible to present a composition of video sources on one or more display devices. The video sources can be video outputting device, for example any external device like an ultra-sound device, a haemodynamic monitoring device (hereinafter also "haemo device'), x-ray device, etc. The video feeds of these video sources can be displayed in the viewports of the display device or devices that the system provides.

The video feeds have certain types, which can be configured appropriate to the application of the system. Examples of such types are ultra-sound, x-ray, haemo, main monitor, auxiliary monitor, etc.

Each such type is associated with a set of display settings, wherein each data within the set represents a property which can influence the way the video feed is presented on the system.

Examples of such properties are a name for identification, an icon for identification, video processing settings, which can control brightness, contrast, etc., a flag or other priority setting, which can indicate to always display a feed, or display a feed on top of all other feeds, position, or size information for the display of the feed, or a configuration that makes the device producing the video feed controllable from the system.

In a common system, these display settings are configured manually for each video input interface by a Field Service Engineer during system installation. If another video source is connected to the system at a later point of time, the display settings for this input interface used by the video source need to be configured manually again.

With a common system, it is therefore important that disconnected devices which are reconnected again use the same video input interface for which it is configured. If a video device is connected to a different video input interface then the incorrect display settings are used and the resulting display of video feeds might become unusable or confusing to the user as some or all of the video feeds would be associated with the wrong name, icon, etc.

Using a system or method according to the invention, it is possible to automatically associate the correct display settings with the video feed, regardless to which video input interface the video source outputting the video feed is connected to. Thus, the possibility that a video source is connected to a video input it was not configured for is eliminated.

An alternative embodiment of the invention may be arranged as a security system, for example a professional security system, automated home security system, etc. Also in this case, video feeds from different cameras may need to be displayed on one or more screens, for example together with an identifier that indicates the location of the camera.

The aforementioned system, the flexible viewing video platform, for example can have 16 video inputs in one version. Many of the video source devices connected thereto, like ultra-sound and haemo, are portable devices that are frequently disconnected and reconnected after use elsewhere. The devices are also disconnected from their video input interface when video cables need to be removed from the floor for cleaning, and may be reconnected to different interfaces afterwards. Also, devices may become disconnected while the system is in use, in which case medical personnel may have to reconnect devices while an intervention is ongoing.

It is therefore not uncommon that a video source device is connected to a video input interface that is not configured for that device and as a result, the corresponding video feed will be display using suboptimal display settings. For example, the video feed will be incorrectly labelled on the system display devices as the label is configured in correspondence with the video input interface only, and not in correspondence with the video feed signal.

As a consequence, a field service engineer will be required to investigate why the video feed from the connected video source cannot be displayed. As a solution, that engineer will have to reconnect the video sources according to the initial setup or to revise the setup information to correspond to the present setup of video source devices.

With the present invention, the video feeds from the video sources are automatically detected by the system. Configuring a video source device for the proper video input interface is no longer required, since the display of the video feeds can be configured with corresponding display settings independently from the video input interface the video source device providing the video feed is connected to.

The automatic recognition of the video feeds greatly improves the flexibility of the video platform system. It no longer matters to which video input interface the device that produces the video feed is connected. Once the video characteristics are made known to the system, the system recognizes the video content and automatically selects the most optimal settings to display the video feed. For instance, the system can configure the icon that identifies the video feed, the optimal video processing settings, if the video feed is mandatory to display, the position and size of the video viewport, and/or device control options (e.g. whether a video source device should receive keyboard and mouse control events).

Thereby less maintenance will be necessary, and video source devices can be connected easier. The video input interfaces are no longer bound to one type of video feeds from video source devices, but video source devices providing any type of video feed can be connected to any video input interface.

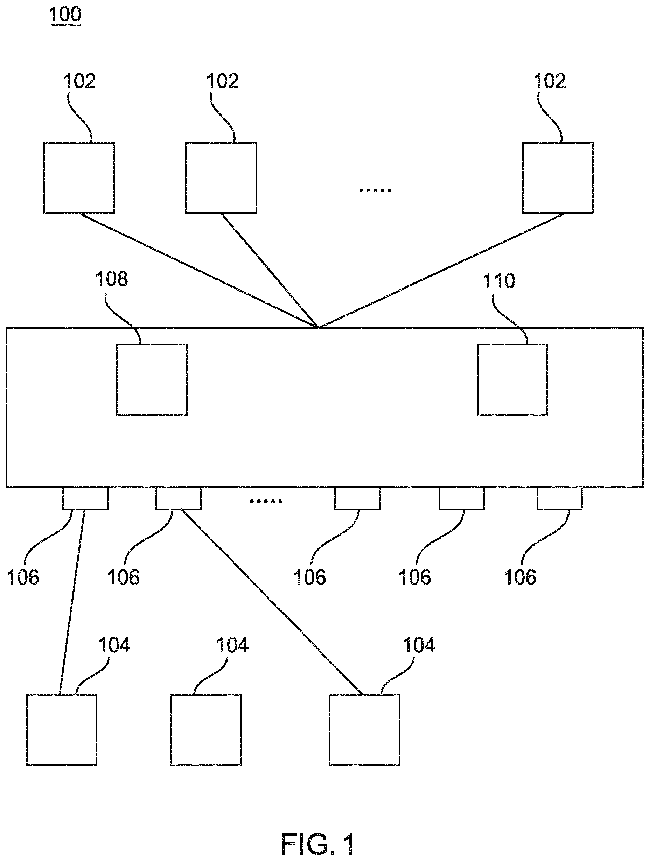

Now turning to Fig. 1, which shows a system 100 for automatic video source detection. The system 100 has one or more display devices 102, which can be arranged according to parameters corresponding to the intended use of the system. The system 100 further has one or more video input interfaces 106, to which one or more video source devices 104 can be connected.

The video feeds which are provided by the video source devices 104 are displayed on the display devices 102 by using display settings.

The system 100 has a memory 108, wherein sets of display settings are stored in correspondence to video feed types.

The processor 110 in the system 100 is configured to recognize the type of video feeds provided by connected video sources based on the contents the respective video feed.

When a type is recognized for a video feed, the corresponding set of display settings from the memory, which is associated with the recognized type of feed, is used to display the video feed on the display devices 102 of the system 100.

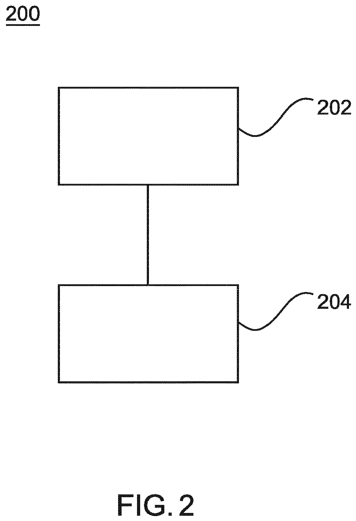

Turning now to Fig. 2, wherein a method 200 for automatic video source detection is shown. Therein, in step 202, a type a video feed of a video source connected to a system via a video input interface, is recognized based on the contents of the video feed. In step 204, display settings which are associated with each type of video feed are applied to the video feed based on the recognized type of the video feed.

Steps 202 and 204 can also be performed for more than one video feed from more than one video source connected to the system via one or more video input interfaces.

In the system of Fig. 1 as well as in the method of Fig. 2 the video input interfaces can make use of existing video interface technology like HDMI, DisplayPort, VGA, etc., but the connection to the video source devices can also be proprietary. Furthermore, the video source devices can be connected wirelessly to one of the interfaces which is a wireless video interface. Wireless connection can also employ an existing technology or can be proprietary. Furthermore, more than one video source devices can be connected to the same interface by means of Daisy Chain technology or a port replicator. Also, more than one video source device can be connected wirelessly to a wireless video input interface.

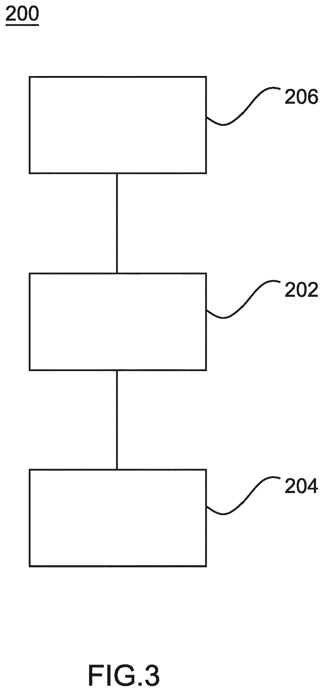

As shown in Fig. 3 for the method 200 in step 206, but not shown for the system 100, the recognition of a video feed can be triggered by the connection event of a video source, i.e. the video source device is connected to one of the video input interfaces.

The recognition can also be triggered by a user request, a hardware request, or a software request. A user request could be entered when a user determines that the display seems to be suboptimal or erroneous. A hardware or software request could be issued by any involved device, if a system change is detected which could possibly interfere with the display of video feeds. Examples of this could be the power adapter recognizing a chance in power supply, a video source device noticing the beginning or end of an operation of one of its visual elements, or a network element noticing a change in the network, like IP address chance, addition, or removal of network elements. The hardware request could even be one of the type to identify an operator using the system such that personal preferences or qualifications to use certain video source devices can be considered. Also, a video source device could recognize that the video provided is different because a different camera is used, which could be done at an interval, or light based, or calendar based, or the like, whereupon the video source device could itself send a hard ware request to recognize its video feed type again and employ the best available settings to the provided feed.

The recognition can also be performed at a predetermined interval like an hour or a day or any other time.

The type of the video feed is recognized based on the contents of the video feed. This can be performed by many different techniques. One example for such a technique is a FLANN image processing algorithm (Fast Approximate Nearest Neighbor Search) to recognize if there are visual elements present in the video feed that are unique to the device producing that video feed.

These unique visual elements can be achieved in several ways, for instance, examples (templates) of the visual elements (e.g. banners or company logo) can be provided for visual elements that are always present in the video feed. That is the type of a video feed can be recognized by looking for visual elements that are always a part of the video feed. These do not need to be in the same position at all times, because a video source device can display a logo at a position where no or the least information is covered by the logo.

Alternatively, the processor 110 or the system performing the method 200 could be configured to automatically learn which visual parts of the video feed remain static over time and use those the identify the type of video feed. For example, a security feed of a living room would always have the doors in the same positions of the video feed, even if furniture is moved.

The video feeds and the visual characteristics of the video feeds can be used to identify the content and thus the type of the video feeds. The processor uses an algorithm and for example can refer to a library of templates with which the processor can compare the identified video characteristics in order to match the video feed to be typed with the template that provides the best match. In order to do this, the processor could allocate a matching score to each of the templates which indicates how good the identified video feed characteristics match the single template. When all templates are compared, the matching scores can be evaluated and the video feed type can be recognized. The video feed can then be properly configured by using the display settings associated with the video feed type, and the system can display the video feed using the optimal settings.

The display settings associated with the video feed types can be for example an image or name for identification, such that the window in which a video feed is displayed can be provided with this image or name, or the control area for that video source device can be marked with that image or name.

The display settings can contain video processing settings, which allow to control brightness, contrast, color, refresh rate or scan rate, or other video processing elements.

The display settings can contain priority settings, which can control the allocation of processing power to the video feed, indicate an "always on top" position for the video feed, restrict user modification options such that a user cannot hide a video feed, cannot resize a video feed window, or provide similar priority based options.

The display settings can contain information regarding position, size, and orientation for the display of the video feed. Thereby crucial information in a medical setting, like patient vital information, can be displayed always in the same position to be accessible without delay from searching the information, or a video feed recognized to be a portrait video feed can be oriented regardless or video pixel dimensions.

Furthermore, the display settings can also contain device control options, indicating whether input control information, via a keyboard, mouse, touchpad, or any other I/O device, should be transmitted to the video source device of a video feed, for example, that it is a movable or zoomable camera, or similar controllable video source device.

In another exemplary embodiment of the present invention, a computer program or a computer program element is provided, that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method 200 of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method 200 as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A system (100) for automatic video source detection, comprising:
- at least one video input interface (106), configured to removably connect at least one video source (104),
- at least one display device (102) for displaying video feeds of different types from the at least one video source,
- a memory (108) comprising at least one set of display settings associated with each type of video feed,
- a processor (110), configured to recognize the type of at least one of the video feeds provided by at least one of the connected video sources based on the contents of the at least one of the video feeds, and to apply display settings to the at least one of the video feeds of the connected video sources based on the recognized type of the at least one of the video feeds.

2. System (100) according to claim 1, wherein the processor (110) is configured to recognize the type of the at least one of the video feeds in response to:
one of the video sources being connected to one of the video input interfaces, a user request, a hardware request, or a software request.

3. System (100) according to one of claims 1 to 2, wherein the processor (110) is configured to recognize the type of video feed by using a FLANN, fast approximate nearest neighbor search, image processing algorithm.

4. System (100) according to one of claims 1 to 3, wherein the display settings comprise at least one of: an image for identification, a name for identification, video processing settings, priority settings, position information, size information, orientation information, and device control options.

5. System (100) according to one of claims 1 to 4, wherein the system is a medical viewing system.

6. A method (200) for automatic video source detection, comprising the steps of:
recognizing (202) a type of at least one video feed of at least one video source connected to a system via at least one video input interface, based on the contents of the at least one of the video feeds, and
applying (204) display settings associated with each type of video feed to the at least one of the video feeds based on the recognized type of the at least one of the video feeds.

7. Method (200) according to claim 6, wherein recognizing (202) is performed in response to one event (206) of one of the video sources is connected to the system, a request of a user of the system, a request of a hardware of the system or connected to the system, or a request of a software on the system.

8. Method (200) according to one of claims 6 to 7, wherein the processor recognizes the type of video feed by using a FLANN, fast approximate nearest neighbor search, image processing algorithm.

9. A computer program element for controlling an apparatus according to one of the claims 1 to 5, which, when being executed by a processing unit, is adapted to perform the method steps of one of the claims 6 to 8.

10. A computer readable medium having stored the program element of claim 9.
